(19) Europäisches Patentamt
European Patent Office
Office européen des brevets

(11) Numéro de publication: **0 345 171 B1**

# (12) FASCICULE DE BREVET EUROPEEN

(45) Date de publication du fascicule du brevet: **13.12.95**

(51) Int. Cl.⁶: **C07D 487/22**, A61K 31/40, //(C07D487/22,257:00,209:00, 209:00,209:00,209:00)

(21) Numéro de dépôt: **89401533.8**

(22) Date de dépôt: **02.06.89**

Le dossier contient des informations techniques présentées postérieurement au dépôt de la demande et ne figurant pas dans le présent fascicule.

## (54) Dérivés de métalloporphyrines, leur préparation, leur application en thérapeutique et leur utilisation pour la préparation de molécules hybrides

(30) Priorité: **02.06.88 FR 8807372**

(43) Date de publication de la demande:
**06.12.89 Bulletin 89/49**

(45) Mention de la délivrance du brevet:
**13.12.95 Bulletin 95/50**

(84) Etats contractants désignés:
**AT BE CH DE ES FR GB GR IT LI LU NL SE**

(56) Documents cités:
**EP-A- 0 118 913**
**EP-A- 0 186 962**
**US-A- 4 386 087**

**Carcinogenesis, 1(11), 889-891 (1980)**

**P. B. Dervan, Science 232, 464-471 (1986)**

**G.B. Dreyer, Proc. Natl. Acad. Sci. USA 82, 968-972 (1985)**

**J. P. Pluka, Science 238, 1129-1132 (1987)**

(73) Titulaire: **CENTRE NATIONAL DE LA RECHERCHE SCIENTIFIOUE**
**15, quai Anatole France**
**F-75007 Paris (FR)**

(72) Inventeur: **Meunier, Bernard**
**Résidence Les Ormes - Bâtiment C3**
**F-31320 Castanet (FR)**
Inventeur: **Etemad-Moghadam, Guita**
**18, rue de Nîmes - Hall 4**
**F-31400 Toulouse (FR)**
Inventeur: **Ding, Li**
**Appartement 99 - 30 Chemin des Maraîchers**
**F-31100 Toulouse (FR)**
Inventeur: **Cros, Suzy**
**7, rue du Coustet**
**F-31520 Ramonville St Agne (FR)**

(74) Mandataire: **Polus, Camille et al**
**c/o Cabinet Lavoix**
**2, Place d'Estienne d'Orves**
**F-75441 Paris Cedex 09 (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

**E. Fouquet, J.Chem. Soc. Chem. Com. 1169-1171 (1987)**

**R. P. Hertzberg, J. Am. Chem. Soc. 104, 313-315 (1982)**

## Description

La présente invention a pour objet de nouvelles molécules hybrides dérivées de métalloporphyrines, leur préparation, leur application en thérapeutique, et leur utilisation pour la préparation de molécules hybrides à usage thérapeutique.

L'utilisation de certains dérivés hématoporphyriniques dans le traitement des cancers par photothérapie a été décrite par exemple par J. Moan (Photobiol. Photochem. 43, 681 (1986). Ces molécules présentent la particularité de s'accumuler au niveau des tumeurs, ce qui permet de les utiliser comme marqueurs biologiques ou agents thérapeutiques. Toutefois, la préparation de ces dérivés conduit souvent à un mélange de produits pas toujours bien définis et de composition variable Cet état de fait justifie le développement de dérivés porphyriniques obtenus par synthèse totale et pouvant se substituer aux hématoporphyrines dans le traitement de cancers par photothérapie.

Par ailleurs, des travaux récents ont montré que des dérivés métallés de porphyrines synthétiques sont capables de couper les acides nucléiques (ADN) in vitro selon un processus d oxydation (EP-A-118 913; E. Fouquet, G. Pratviel, J. Bernadou et B. Meunier, J. Chem. Soc., Chem. Commun., 1169 (1987). Cette activité nucléase de métalloporphyrines hydrosolubles est d'autant plus intéressante qu'elle est observable pour des concentrations très faibles de réactifs (de l'ordre de $10^{-6}$ à $10^{-8}$ M), inférieures à celles de la bléomycine (un antibiotique antitumoral possédant une forte activité nucléase en présence d'ions ferriques, d'oxygène moléculaire et de réducteurs). Les nombreuses études menées ces dernières années sur la pharmacologie moléculaire de ce médicament majeur de la chimiothérapie anticancéreuse indiquent que son activité cytotoxique et antitumorale peut être due à sa capacité à dégrader l'ADN de cellules tumorales.

Selon la présente invention on a effectué la synthèse de molécules hybrides dérivées de métalloporphyrines (dont la sphère de coordination autour d'ions fer ou manganèse modélise l'arrangement des ligands peptidiques de la bléomycine) ayant les deux propriétés suivantes :

- activité nucléase sur l'ADN in vitro, et
- activité cytotoxique sur cellules entières,

qui, à la connaissance des inventeurs, n'ont pas encore été décrites. L'invention concerne donc de nouveaux dérivés porphyriniques possédant cette double propriété et résultant du couplage d'une entité possédant une affinité pour les acides nucléiques et capable aussi de moduler l'activité biologique de ces métalloporphyrines cytotoxiques (polyamines, polylysine, oligonucléotides...) avec un dérivé de metalloporphyrine.

L'invention a pour objet une molécule hybride résultant du couplage d'un vecteur présentant une affinité pour les acides nucléiques -choisi parmi la 9-méthoxy-ellipticine, les oligonucléotides, oligopeptides, protéines et fragments de protéines - et d'un dérivé de métalloporphyrine de formule générale

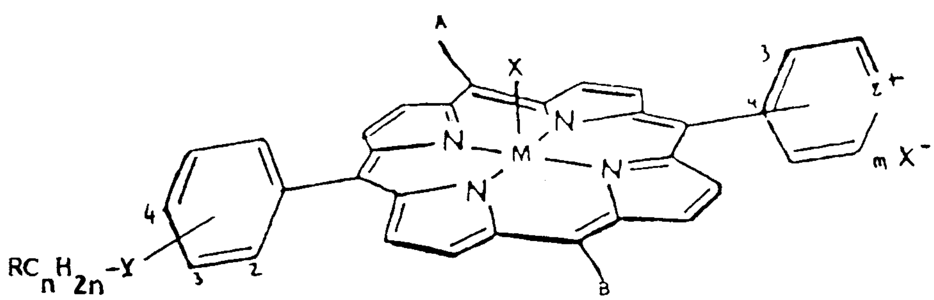

I

dans laquelle A et B représentent chacun

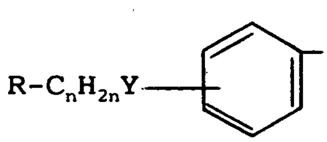

ou

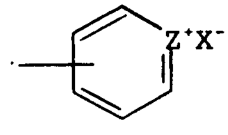

et $Z^+$ représente $N^+-R_1$ ou $C-N^+R_1R_2R_3$, $R_1$ étant un groupe aliphatique droit ou ramifié en $C_1$ à $C_{10}$, et $R_2$ et $R_3$ étant chacun un atome d'hydrogène ou un groupe aliphatique droit ou ramifié en $C_1$ à $C_{10}$, R représente un groupe $NH_2$, OH, COOH ou $-N(R_1)^+{}_3$ ou un atome d'halogène,

n est 0 ou un entier de 1 à 10, le groupe alkylène correspondant pouvant être droit ou ramifié,
M représente Mn,
$X^-$ représente l'anion d'un acide carboxylique pharmaceutiquement acceptable, m étant un entier de 1 à 5,
et Y représente une liaison ou, lorsque n est différent de 0, un radical -O-, -CO- ou -CONH-.

De préférence, $R_1$ représente un groupe alkyle, en particulier méthyle ou éthyle, et $R_2$ et $R_3$ représentent chacun un atome d'hydrogène ou méthyle.
X- est choisi notamment parmi les anions des acides carboxyliques solubles couramment utilisés en pharmacie, en particulier : acétate, propionate, butyrate ascorbate, benzoate, cinnamate, citrate, fumarate, glycolate, malonate, tartrate, malate, maléate et mandélate.

De préférence encore $Z^+$ représente le groupe $N^+$-$R_1$.

Les composés de formule I peuvent être préparés par

(a) condensation d'un phényl-carboxaldéhyde de formule

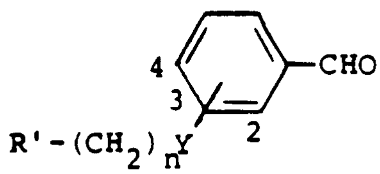

II

dans laquelle n et Y ont la même signification que dans la formule I et R' est le groupe R éventuellement protégé de la formule I,
et d'un aldéhyde de formule

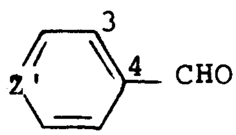

III

dans laquelle Z' représente N ou

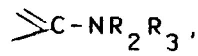

$R_2$ et $R_3$ ont la même signification que dans la formule I,
avec le pyrrole en milieu acide, de façon à obtenir une porphyrine (PP) tétrasubstituée en positions 5,10, 15,20 de formule générale

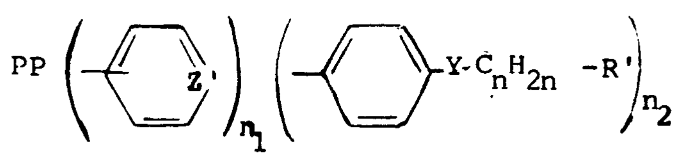

IV

dans laquelle R', n Y et Z' ont les mêmes significations que dans les formules II et III respectivement, et $n_1$ et $n_2$ sont des entiers de 1 à 3 avec $n_1 + n_2 = 4$,
b) le cas échéant le composé IV est soumis à une déprotection du radical R' pour obtenir une porphyrine tétrasubstituée en 5,10,15,20 de formule générale

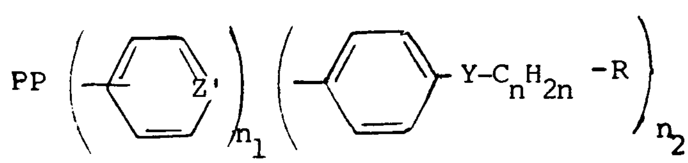

V

dans laquelle Z' a la même signification que dans la formule III, PP, $n_1$ et $n_2$ sont comme dans la formule IV et n, R, et Y ont la même signification que dans la formule I,

c) le composé de formule V, après protection éventuelle de la fonction R, est soumis à une réaction d'alkylation à l'aide d'un halogènure $R_1$-hal, $R_1$ ayant la même signification que dans la formule I et hal désignant le brome ou d'iode, de manière à obtenir une porphyrine tétrasubstituée en 5,10,15,20 de formule générale

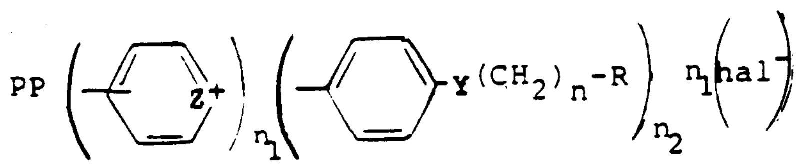

VI

et

d) ce composé est métallée à l'aide d'un sel MXp, M et X ayant la même signification que dans la formule I et p correspondant à la valence du métal M.

L'étape a) est effectuée en milieu acide, notamment acide propionique au reflux, en présence d'anhydride acétique.

Selon la signification du radical R, une protection peut être nécessaire. Si R = $NH_2$, on choisira R' = $NO_2$ et alors l'étape b) consistera à réduire $NO_2$ en $NH_2$, notamment par le chlorure stanneux en milieu acide fort. Si R = COOH, on choisira pour R' un ester d'alkyle correspondant et l'étape b) consistera à hydrolyser cet ester.

L'étape c) d'alkylation est effectuée de manière classique, dans un solvant polaire neutre tel que le diméthylformamide (DMF) au reflux.

L'étape d) de métallation, peut aussi être effectuée dans le DMF au reflux, notamment en présence de 2,4,6-collidine.

Selon une variante, les étapes c) et d) peuvent être inversées.

Les molécules hybrides de l'invention résultent du couplage d'un composé de formule I avec un vecteur présentant une affinité pour les acides nucléiques -choisi parmi la 9-méthoxy-ellipticine, les oligonucléotides, oligopeptides, protéines et fragments de protéines.

Les molécules hybrides particulières de l'invention sont celles dans lesquelles le dérivé de métalloporphyrine est relié audit vecteur par une fonction amine ou alcool greffée sur un groupe phényle de la métalloporphyrine.

Comme molécules préférées, on citera celles dans lesquelles le vecteur est la 9-méthoxy-ellipticine.

Les exemples suivants illustrent l'invention.

Les spectres de résonance magnétique nucléaire ont été obtenus sur un appareil Bruker 250 WM FT. Les spectres de masse ont été obtenus soit sur un appareil Ribermag R1010 pour les spectres en DCI ($NH_3$), Varian Mat 311A en désorption de champ ou ZAB pour les spectres en FAB et les spectres UV-visible sur un appareil Varian-Cary 2300.

EXEMPLE 1 : Pentaacétate de 5-(4-aminophényl)-10,15, 20-tris(N-méthyl-4-pyridyl)-porphyrine de manganèse (III) et dérivés apparentés.

a) 5-(4-nitro-phényl)-10,15,20-tris(4-pyridyl)-porphyrine

Un mélange de 3 g (0,020 mole, 1,75 eq.) de nitro-4-phényl carboxaldéhyde dans 158 ml d'acide propionique et 8 ml d'anhydride acétique est chauffé à 110 °C sous agitation. A cette solution, on ajoute lentement 3,2 ml (0,034 mole, 3 eq.) de pyridine-4-carboxaldéhyde puis 3,1 ml (0,045 mode, 4 eq.) de pyrrole et on porte le mélange au reflux pendant 1 h 30. Revenu à la température ambiante, le mélange est évaporé à sec neutralisé avec une solution aqueuse d'ammoniaque. Après filtration le précipité est lavé plusieurs fois avec du dichlorométhane. La phase organique est ensuite concentrée puis purifiée sur uné colonne de silice sèche (éluant : dichlorométhane puis dichlorométhane/éthanol : 95/5). Produit isolé = 0,526 g ; Rdt = 7,1 %.

CCM : Rf = 0,51 (éluant : $CH_2Cl_2$/EtOH 95/5).

UV-visible ($CHCl_3$) $\lambda$ ($\epsilon$) : 640 (1,1 x $10^3$); 586 (2,0 x $10^3$); 546 (2,8 x $10^3$); 512 (6,7 x $10^3$); 418 (1,6 x $10^5$) (bande de Soret).

RMN 1H ($CDCl_3$); $\delta$ : 9,06 (d, 6H, J = 5,9 Hz, protons 2,6-pyridine); 8,87 (m, 6H, $\beta$-pyrrole); 8,81 (d,

2H, J = 4,9 Hz, β-pyrrole);; 8,66 (d, 2H, J = 8,60 Hz, protons 2,6-nitro-4-phényl); 8,38 (d, 2H, J = 8,60 Hz, protons 3,5-nitro-4-phényl); 8,15 (d, 6H, J = 5,9 Hz protons 3,5-pyridine); -2,89 (s, 2H, NH pyrrole).

Analyse : (dans les calculs on a considéré qu'il y avait une molécule d'eau de solvatation), $C_{41}H_{26}N_8O_2$, $H_2O$.

| | C | H | N |
|---|---|---|---|
| Calculé % | 72,34 | 4,15 | 16,46 |
| Trouvé % | 72,87 | 4,06 | 16,93 |

b) 5-(4-amino-phényl)-10,15,20-tris(4-pyridyl)-porphyrine

A 0,252 g (0,38 mmole) de la porphyrine (a) dans 18 ml d'acide chlorhydrique 6N, on ajoute 0,672 g (3 mmoles, 8 eq.) de chlorure stanneux dihydraté et on laisse le mélange sous agitation à température ambiante pendant 15 h. Le mélange est ensuite neutralisé avec de la soude 1 N puis extrait au dichlorométhane. La phase organique est lavée à l'eau puis séchée sur sulfate de magnésium. Le solvant est évaporé à sec. Le résidu repris dans du dichlorométhane est précipité par l'addition de l'hexane ($CH_2Cl_2$/hexane : 1/10) puis filtré et séché sous vide.

Produit isolé = 0,21 g: Rdt = 87 %.

CCM : Rf = 0,23 (éluant :$CH_2Cl_2$/EtOH 95/5)

UV-visible ($CHCl_3$) λ (ε) : 652 (3,0 x $10^3$); 590 (3,2 x $10^3$); 554 (4,5 x $10^3$); 516 (7,8 x $10^3$); 420 (8,8 x $10^4$) (bande de Soret).

Masse (DCI) : $M^+$ = 633 et (FD) : $M^+$-H = 632

RMN $^1$H (DMSOd6 à 303 K) δ : 9,15 (d, 6H, J = 5,50 Hz, p-2,6 pyridine); 8,98 (m, 8H, β-pyrrole); 8,36 (d, 6H, J = 5,50 Hz, p-3,5-pyridine); 7,99 (d, 2H, J = 8,20 Hz, p-2,6-amino-4-phényl); 7,14 (d, 2H, J = 8,20 Hz, p-3,5-amino-4-phenyl); 5,87 (s, 2H, $NH_2$);-2,80 (s, 2H, NH pyrrole).

c) 5-(4-benzoyl-amino-phényl)-10,15,20-tris(4-pyridyl)-porphyrine

0,010 ml (0,086 mmole, 1,8 eq.) de chlorure de benzoyle est ajouté goutte à goutte à une solution de 0,030 g (0,047 mmole) de la porphyrine (b) dans 3 ml de pyridine anhydre maintenue dans un bain de glace. Le mélange est agité pendant 1 h à 0° C. Au bout de ce temps, le solvant est évaporé à sec et le résidu est repris dans du dichlorométhane. Cette solution est lavée à l'eau distillée, avec de la soude à 5% puis à l'eau distillée. La phase organique est séchée sur sulfate de magnésium puis évaporée à sec. Le résidu est séché sous vide.

Produit isolé = 0,025 g ; Rdt = 71 %.

RMN $^1$H ($DMSOd_6$ à 297 K) δ 10,72(s, 1H, NH) ; 8,96 (d, 6H, J = 5,3 Hz, protons 2,6 pyridine) ; 8,93 (d, 2H, J = 4,7 Hz, β-pyrrole) ; 8,83 (s, 4H, β-pyrrole) ; 8,82 (d, 2H, J = 4,7 Hz, β-pyrrole) ; 8,22 (m, 10H, protons 3,5 pyridine et phényl); 8,11 (d, 2H, J = 8,0 Hz, protons 2,6 amino-4-phényl) ; 7,65 (m, 3H, protons 3,5 amino-4-phényl et phényl-para) ; - 2,85 (s, 2H, NH pyrrole).

*d) Pentaacétate de 5-(4-amino-phényl)-10,15,20-tris(N-méthyl-4-pyridyl)-porphyrine de manganèse(III).*

A 0,030 g (0,05 mmole de porphyrine (c) dans 3 ml de DMF, on ajoute 0,062 ml (0,047 mmole, 10 eq.) de 2,4,6 collidine puis 0,047 mmole (10 eq.) de sel de manganèse, de fer, de zinc ou de nickel. Le mélange est porté au reflux pendant 3 heures puis on le laisse sous agitation à température ambiante pendant 15 heures. Le solvant est évaporé à sec et le résidu est lavé à l'eau puis, purifié sur se colonne sèche d'alumine neutre (éluant : dichlorométhane puis dichlorométhane / éthanol). A la métalloporphyrine ainsi obtenue dans 3 ml de DMF, on ajoute l'iodure de méthyle (10 eq.) et le mélange est agité à la température ambiante pendant 15 heures, Le mélange est ensuite évaporé à sec. La déprotection du groupement amine se fait par addition d'une solution d'ammoniaque 9 M. Le mélange est alors agité à 70°C pendant 3h puis 15h à température ambiante. Au bout de ce temps, le mélange est évaporé à sec puis traité à l'acide chlorhydrique 6M, évaporé à sec et repris dans du méthanol.

L'addition de résine essorée d'échangeurs d'ions de type Amberlite IRN 78 sous forme acétate (4 eq.) à cette solution suivie d'une agitation de 3 heures à la température ambiante permet, après filtration puis évaporation du solvant, d'obtenir l'acétate du titre. Le produit est ensuite purifié par trituration avec de

l'éther.

On a obtenu de la même manière le dérivé de fer, de zinc et de nickel correspondant en remplaçant l'acétate de manganèse($Mn(CH_3COO)_2,4H_2O$)respectivement par $FeCl_2$, $4H_2O$; $Zn(CH_3COO)_2$, $2H_2O$ ou $NiCl_2$, $6H_2O$ (10 eq. chaque fois), Rendements de 60 à 70%.

Dérivé du manganèse: UV-visible ($H_2O$ à 7,73 x $10^{-6}$ M) λ (ε) 596 (3,9 x $10^3$); 554 (8,6 x $10^3$); 464(7,5 x $10^3$).

Dérivé du zinc: UV-visible ($H_2O$ à 23,9 x $10^{-6}$ M) λ (ε) 606 (2,5 x $10^3$) 562 (5,0 x $10^3$) 432 (5,8 x $10^4$).

Dérivé du nickel: UV-visible ($H_2O$ à 14,3 x $10^{-6}$ M) λ (ε) 568 (4,9 x $10^3$) 530 (1,0 x $10^4$) 417 (1,1 x $10^5$).

*e) Variante de préparation du triacétate de 5-(4-amino-phényl)-10,15,20-tris(N-méthyl-4-pyridyl)-porphyrine par méthylation de la porphyrine (a) suivie d'une réduction de la fonction nitro.*

A 0,016 g (0,024 mmole) de porphyrine (a) dans 20 ml de DMF, on ajoute un large excès d'iodure de méthyle (1 ml). Le mélange est agité à 40° C pendant 2 h puis le solvant est évaporé à sec. Le résidu est repris dans 3 ml d'acide chlorhydrique 6N. Après addition du chlorure stanneux dihydraté, l'agitation est maintenue à température ambiante pendant 15 h. Le solvant est ensuite évaporé à sec. Le résidu est repris dans du méthanol.

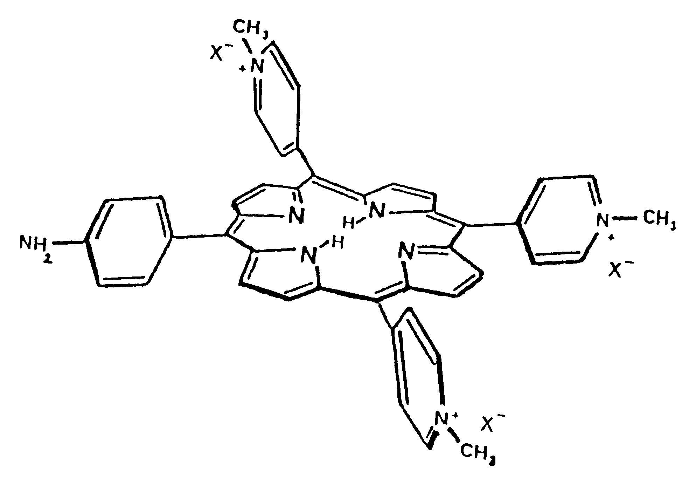

L'addition de résine essorée d'échangeurs d'ions de type Amberlite IRN 78, sous forme acétate (4 eq.) à cette solution, suivie d'une agitation de 3 h à la température ambiante, permet, après filtration puis évaporation du solvant, d'obtenir l'acétate correspondant. Le résidu est repris dans de l'eau et après un passage sur une colonne LH20, la porphyrine est purifiée. Rdt = 83%. Sa formule développée (X = $CH_3COO$) est représentée sur la figure annexée

RMN $^1H$ ($CD_3COOD$) δ 9,51 (m, 6H, protons 2,6 pyridine); 9,04 (m large, 14H, β-pyrrole et protons 3,5 pyridine); 8,35 (m, 2H, protons 2,6 amino-4-phényl); 7,80 (m, 2H, protons 3,5 amino-4-phényl); 4,92 (m, 9H, N-méthyl-4-pyridyl).

f) Iodure de 5-(4-triméthyl-amino-phényl)-10,15,20-tris(N-méthyl-4-pyridyl) porphyrine

A 0,014 g (0,02 mmole) de porphyrine (b) dans 4 ml de DMF, on ajoute goutte à goutte 0,014 ml (0,2 mmole, 10 eq.) d'iodure de méthyle et on porte le mélange au reflux pendant 3 heures. La solution est ensuite agitée à la température ambiante pendant 15 heures puis le solvant est évaporé à sec. Le résidu est trituré avec du dichlorométhane, du méthanol puis de l'acétone. Après filtration, le produit est séché sous vide. Produit isolé = 0,019 g: Rdt = 83%.

UV-visible (MeOH/AcOH): 99/1) λ (ε) : 655 (1,3 x $10^3$); 590 (4 x $10^3$); 560 (4 x $10^3$); 515 (1,3 x $10^4$); 421 (7,0 x $10^4$) (bande de Soret).

Masse (FD) : m/e = 660

RMN$^1H$ (DMSO-d6 à 303 K) δ : 9,60 (d, 6H, J = 5,9 Hz, p-2,6-pyridine); 9,27 (m, 8H,β-pyrrole); 9,13 (d, 6H, J = 5,9 Hz, p-3,5-pyridine); 8,18 (d, 2H, J = 8,6 Hz, p-2,6-amino-4-phényl); 7,34 (d, 2H, J = 8,6 Hz, p-3,5-amino-4-phényl); 4,86 (s large, 9H, N-méthyl-4-pyridyl), 3,46 (s large, 9H, $N(CH_3)_3$; -2,73 (s, NH pyrrole).

g) Pentaacétate de 5-(4-trimethyl-aminophényl)-10, 15,20-tris (N-méthyl-4-pyridyl)-porphyrine de manganèse (III)

A 0,053 g (0,05 mmole) de iodure de porphyrine (f) dans 4 ml de DMF, on ajoute 0,062 ml (0,047 mmole, 10 eq.) de 2,4,6-collidine puis 0,047 mmole (10 eq.) d'acétate de manganèse, Mn $(CH_3COO)_2$, $4H_2O$. Le mélange est porté au reflux pendant 3 heures puis on le laisse sous agitation à température ambiante pendant 15 heures. Le solvant est évaporé à sec et le résidu est lavé a l'eau puis repris dans du méthanol. L'addition de résine essorée d'échangeurs d'ions de type Amberlite IRN 78 sous forme acétate (4 eq.) à cette solution suivie d une agitation de 3 heures à la température ambiante permet, après filtration puis évaporation du solvant, d'obtenir l'acétate du titre. Le produit est ensuite purifié par précipitation dans un mélange méthanol/acétone, Rdt = 80%. P.F. supérieure à 240° C.

h) Variante de préparation par inversion des étapes f) et g) précédentes.

A 0,030 g (0,05 mmole de porphyrine (c) déprotégée dans 3 ml de DMF, on ajoute 0,062 ml (0,047 mmole, 10 eq.) de 2,4,6 collidine puis 0,047 mmole (10 eq.) de sel de manganèse, (Mn $CH_3COO)_2$, $4H_2O$,. Le mélange est porté au reflux pendant 3 heures puis on le laisse sous agitation à température ambiante

pendant 15 heures. Le solvant est évaporé à sec et le résidu est lavé à l'eau puis, purifié sur une colonne sèche d'alumine neutre (éluant : dichlorométhane puis dichlorométhane/éthanol). A la métalloporphyrine ainsi obtenue dans 3 ml de DMF, on ajoute l'iodure de méthyle (10 eq) et on porte le mélange au reflux pendant 4 heures puis, on le laisse agiter à la température ambiante pendant 15 heures. Le mélange est ensuite évaporé à sec puis repris dons du méthanol. L'addition de résine essorée d'échangeurs d'ions de type Amberlite IRN 78 sous forme acétate (4 eq) à cette solution suivie d'une agitation de 3 heures à la température ambiante permet, après filtration puis évaporation du solvant, d'obtenir l'acétate du titre. Le produit est ensuite purifié par précipitation dans un mélange méthanol/acétone. Rdt = 82 %.

EXEMPLE 2 : Dérivés hydroxyphényliques de porphyrine.

*i) 5-(4-hydroxyphényl)-10,15,20-tris(4-pyridyl)-porphyrine*

Un mélange de 3,19 g (0,026 mole; 1,75 eq) de hydroxy-4-phenylcarboxaldéhyde dans 205 ml d'acide propionique et 10 ml d'anhydride acétique est chauffé à 110 °C sous agitation. A cette solution, on ajoute lentement 4,27 ml (0,045 mole; 3 eq) de pyridine-4-carboxaldéhyde, puis 4,13 ml (0,06 mole ; 4 eq) de pyrrole et on porte le mélange au reflux pendant 1 h 30. Revenu à la température ambiante, le mélange est évaporé à sec et neutralisé avec une solution aqueuse d'ammoniaque. Après filtration, le précipité est lavé plusieurs fois avec du dichlorométhane. La phase organique est ensuite concentrée puis purifiée sur une colonne de silice sèche (éluant: dichlorométhane/éthanol 95/5). Produit isolé = 0,52 g ; Rdt = 5,5 %; ccm: $R_f$ = 0,14 (éluant: $CH_2Cl_2$/éthanol, 95/5).

UV-visible ($CHCl_3$ à 7,2 x $10^{-6}$ M) λ (ε) 642 (3,2 x $10^3$) ; 587 (7,9 x $10^3$) ; 545 (9,4 x$10^3$) ; 511 (2,8 x $10^4$) ; 416 (5,1 x $10^5$)(bande de Soret).

RMN$^1$H ($CDCl_3$ à 294 K) δ 9,04 (d, 6H, J = 5,2 Hz, protons 2,6-pyridine) ; 8,95 (d, 2H, J = 4,8 Hz, β-pyrrole) ; 8,86 (m, 6H, β-pyrrole); 8,20 (d, 2H, J=8,3 Hz, protons 2,6-phénoxy-4) ; 8,16 (d, 6H, J = 5,5 Hz, protons 3,5-pyridine) ; 7,52 (d, 2H, J = 8,3 Hz, protons 3,5-phénoxy-4) ; 2,80 (q, 2H, J = 7,5 Hz, $\underline{CH_2}CH_3$) ; 2,50 (m, 2H, OH); 1,42 (t, 3H, J = 7,5 Hz, $\underline{CH_3}CH_2$) ; -2,89 (s, 2H, NH pyrrole).

Analyse : dans les calculs, on a considéré également une molécule d'éthanol de solvatation, $C_{41}H_{27}N_7O$, EtOH.

| | | | |
|---|---|---|---|
| Calculé % : | C, 75,96 ; | H, 4,97 ; | N, 14,43. |
| Trouvé % : | C, 76,08 ; | H, 4,77 ; | N, 13,09. |

*j) Cis-5,10-bis(4-hydroxy-phényl)-15,20-bis(4-pyridyl)-porphyrine*

Lors de la purification sur colonne de silice sèche du mélange réactionnel du paragraphe précédent nous obtenons également la cis-5,10-bis(4-hydroxy-phenyl)-15,20-bis(4-pyridyl) -porphyrine (éluant : $CH_2Cl_2$/EtOH, 97,5/2,5) : produit isolé = 0,74 g ; Rdt = 7,7% ; ccm : $R_f$ = 0,21 (éluant : $CH_2Cl_2$/EtOH, 95/5).

UV-visible (dans $CHCl_3$ à 7,2 x $10^{-6}$ M) λ (ε) 642 (2,1 x $10^3$) ; 586 (4,4 x $10^3$) ; 546 (5,8 x$10^3$) ; 512 (1,5 x $10^4$) ; 415 (3 x $10^5$ (bande de Soret).

RMN$^1$H ($CDCl_3$) δ 9,03 (d, 4H, J = 5,8 Hz, protons 2,6-pyridine) ; 8,94 (d, 2H, J = 4,9 Hz, β-pyrrole) ; 8,91 (s, 2H, β-pyrrole) ; 8,84 (s, 2H, β-pyrrole) ; 8,81 (d, 2H, J = 4,9 Hz, β-pyrrole) ; 8,20 (d, 4H, J = 8,5 Hz, protons 2,6-phénoxy-4) ; 8,17 (d, 4H, J = 5,8 Hz, protons 3,5-pyridine) ; 7,51 (d, 4H, J = 8,5 Hz, protons 3,5-phénoxy-4) ; 2,80 (q, 2H, J = 7,5 Hz, $\underline{CH_2}CH_3$) ; 1,42 (t, 3H, J = 7,5 Hz, $\underline{CH_3}CH_2$) ; -2,88 (s, 2H, NH pyrrole).

Analyse : dans les calculs, on a considéré qu'il y avait 1,75 molécules d'éthanol de solvatation, $C_{42}H_{28}N_6O_2$, 1,75 EtOH.

| | | | |
|---|---|---|---|
| Calculé % : | C, 74,95 ; | H, 5,28 ; | N, 11,53. |
| Trouvé % : | C, 74,33 ; | H, 4,99 ; | N, 11,23. |

*k) 5-(4-pyridyl)-10,15,20-tris (4-hydroxy-phényl)-porphyrine*

De la même manière que précédemment, nous obtenons la 5-(4-pyridyl)-10,15,20-tris (4-hydroxy-phényl)-porphyrine (éluant : dichlorométhane). Produit isolé = 0,62 g ; Rdt = 6,3 % ; ccm : $R_f$ = 0,31 (éluant : $CH_2Cl_2$/EtOH 95/5).

UV-visible (dans $CHCl_3$ à 1,2 x $10^{-5}$ M) λ (ε) 642 (2,6 x $10^3$) ; 587 (4,4 x $10^3$) ; 548 (5,5 x $10^3$) ; 512 (1,3 x $10^4$) ; 416 (3,3 x $10^5$) (bande de Soret).

RMN$^1$H ($CDCl_3$) δ 9,02 (d, 2H, J = 5,9 Hz, protons 2,6-pyridine) ; 8,91 (d, 2H, J = 4,9 Hz, β-pyrrole) ; 8,89 (s, 4H, β-pyrrole) ; 8,79 (d, 2H, J = 4,9 Hz, β-pyrrole); 8,20 (d, 6H, J = 8,4 Hz, protons 2,6-phénoxy-4) ; 8,16 (d, 2H, J = 5,9 Hz, protons 3,5-pyridine) ; 7,51 (d, 6H, J = 8,4 Hz, protons 3,5-phénoxy-4) ; 2,79 (q, 2H, J = 7,5 Hz, $\underline{CH_2}CH_3$) ; 1,58 (m, 6H, OH) ; 1,42 (t, 3H, J = 7,5 Hz, $\underline{CH_3}CH_2$) ; -2,86 (s, 2H, NH pyrrole).

Analyse : dans les calculs, on a considéré qu'il y avait deux molécules d'éthanol de solvatation, $C_{43}H_{29}N_5O_3$, 2EtOH.

| | | | |
|---|---|---|---|
| Calculé % : | C, 74,67 : | H, 5,47 : | N, 9,27. |
| Trouvé % : | C, 74,49 : | H, 5,06 : | N, 8,45. |

*l) Acétate de 5-(4-hydroxyphényl)-10,15,20-tris(4-pyridyl)-porphyrine de manganèse.*

La réaction de métallation s'effectue selon le même mode opératoire décrit pour la porphyrine (g) de l'exemple 1.

Masse ($FAB^+$) : m/e 687 ($M^+$)

*m) Triacétate de 5-(4-hydroxyphényl)-10,15,20-tris (N-méthyl-4-pyridyl)-porphyrine.*

La méthylation de la porphyrine de l'exemple (i) par l'iodure de méthyle dans du DMF suivie d'une réaction d'échange d'ions iodure en acétate se fait selon la méthode décrite dans l'exemple (f). (Rdt = 88 %).

RMN$^1$H ($CD_3COOD$) δ 9,46 (m, 6H, protons 2,6-pyridine) ; 9,35 (m, 2H, β-pyrrole) ; 9,14 (m, 4H, β-pyrrole) ; 8,99 (m, 6H, protons 3,5-pyridine) ; 8,71 (m, 2H, β-pyrrole) ; 8,15 (d, 2H, J = 7,6 Hz, protons 2,6-phénoxy-4) ; 7,56 (d, 2H, J = 7,6 Hz, protons 3,5-phénoxy-4) ; 4,91 (s large, 9H, N-Me).

*n) Tétraacétate de 5-(4-hydroxyphényl)-10,15,20-tris (N-méthyl-4-pyridyl)-porphyrine de manganèse (III).*

La métallation de la porphyrine (i) avec de l'acétate de manganèse suivie d'une réaction de méthylation par l'iodure de méthyle se font dans les mêmes conditions que pour l'exemple 1(h) et aprés l'échange des contre-ions iodure en acétate sur une résine échangeuse d'ions, on obtient le produit attendu.

Produit isolé = 0,018 g ; Rdt = 76 %.

UV-visible (dans $H_2O$ à 6,1 x $10^{-6}$ M) λ (ε) 600 (2,9 x $10^3$) ; 560 (6,6 x $10^3$) ; 464 (7,3 x $10^4$) (bande de Soret).

EXEMPLE 3 : Dérivés amino-propyloxyphényliques de porphyrine.

*o) 5-[4-(3-amino-propyloxy)-phényl)-10,15,20-tris(4-pyridyl)-porphyrine.*

A une solution de 0,051 g (0,8 mmole) de la porphyrine de l'exemple (i) dans 3 ml de DMF sont ajoutés à température ambiante 0,65 g (16,4 mmole : 20,5 eq.) de soude pilée. La couleur de la solution passe de violet au vert et le mélange est agité pendant 15 minutes. 0,017 g (0,88 mmole ; 1,1 eq) de bromohydrate de brome-3-propylamine sont ajoutés à cette solution et l'agitation est maintenue pendant 3 h. L'avancement de la réaction est suivie par ccm (plaque de silice ; éluant : $CH_2Cl_2$ / EtOH, 50/50). Au bout de ce temps, 0,017 g de bromohydrate de bromo-3-propylamine sont rajoutés et le mélange est encore agité pendant 2 h. On ajoute alors 2 ml de méthanol et 5 ml d'eau distillée au mélange réactionnel. La solution obtenue est extraite dichlorométhane. La phase organique est lavée à l'eau distillée (2 fois), séchée sur sulfate de sodium et évaporée à sec. Le produit est purifié par chromatographie sur colonne sèche

d'alumine basique (éluant : méthanol / acide acétique 80/20); produit isolé = 0,095 g ; Rdt = 85 %. ccm : $R_f$ = 0,29 (éluant : $CH_2Cl_2$/EtOH, 80/20 avec 1 % de $NH_4OH$).

UV-visible (dans MeOH à 1,33 x $10^{-5}$ M) λ (ε) 642 (1,6 x $10^3$) ; 583 (3,1 x $10^3$) ; 544 (3,8 x $10^3$) ; 510 (9,8 x $10^3$) ; 412 (1,8 x $10^5$) (bande de Soret).

RMN $^1$H ($MeOHd_4$ à 294 K) δ 8,98 (d, 6H, J = 5,5 Hz, protons 2,6-pyridine) ; 8,97 (m, 8H, β-pyrrole) ; 8,25 (d, 6H, J = 5,5 Hz, protons 3,5-pyridine) ; 8,18 (d, 2H, J = 8,5 Hz, protons 2,6-phenoxy-4); 7,47 (d, 2H, J = 8,5 Hz, protons 3,5-phenoxy-4) ; 5,0 (m, 2H, $NH_2$) ; 4,53 (t, 2H, J = 5,6 Hz, $OCH_2$) ; 3,4 (m, 2H, $CH_2N$) ; 2,45 (quintuplet, 2H, J = 5,5 Hz, $CH_2$). Masse (DCI / $NH_3$) : m/e = 692 ($M^+$ + H); fragments : 634 ($M^+$- $(CH_2)_3NH_2$).

*p) Cis-5,10-bis[4-(3-amino-propyloxy)-phenyl]-15,20-bis(4-pyridyl)-porphyrine.*

En poursuivant le même mode opératoire que celui décrit dans l'exemple (o) mais à partir de la porphyrine (j), nous obtenons la porphyrine dipyridyle porteuse de 2 bras en position cis. Produit isolé = 0,066 g ; Rdt = 79 % ; ccm : $R_f$ = 0 (éluant : $CH_2Cl_2$ / EtOH, 80/20).

UV-visible (dans MeOH/$CHCl_3$, 95/5 à 9,2 x $10^{-6}$ M) λ (ε) 644 (2,2 x $10^3$) ; 588 (3,3 x $10^3$) ; 548 (4,9 x $10^3$) ; 512 (9,2 x $10^3$) ; 414 (1,8 x $10^7$) (bande de Soret).

RMN $^1$H ($CDCl_3$) δ 9,01 (d, 4H, J = 5,8 Hz, protons 2,6-pyridine) 8,92 (d, 2H, J = 4,9 Hz, β-pyrrole) ; 8,89 (s, 2H, β-pyrrole) ; 8,81 (s 2H, β-pyrrole) ; 8,77 (d, 2H, J = 4,9 Hz, β-pyrrole) ; 8,14 (d, 4H, J = 5,8 Hz, protons 3,5-pyridine) ; 8,08 (d, 4H, J = 8,4 Hz, protons 2,6-phenoxy-4) ; 7,27 (d, 4H, J = 8,4 Hz, protons 3,5-phenoxy-4) ; 4,32 (t, 4H, J = 5,9 Hz, $OCH_2$) ; 3,08 (t, 4H, J = 6,0 Hz, $CH_2N$) ; 2,32 (m, 4H, $CH_2$) ; - 2,84 (s, 2H, NH pyrrole).

Masse (DCI) : m/e 765 ($M^+$ +2) ; fragments à 707 et 649.

Analyse : dans les calculs, on a considéré qu'il y avait 3 molécules d'éthanol de solvatation, $C_{48}H_{42}N_8O_2$, 2,5EtOH.

| | | | |
|---|---|---|---|
| Calculé % : | C, 72,48 : | H, 6,55 : | N, 12,76. |
| Trouvé % : | C, 72,00 : | H, 6,67 : | N, 12,44. |

*q) Tétraacétate de 5-[4-(N-triméthyl-3-aminopropyloxy)phenyl]-10,15,20-tris (N-méthyl-4-pyridyl)-porphyrine*

La méthylation de la porphyrine (i) par la méthode déjà décrite dans l'exemple (1f) suivie d'un échange des contre-ions iodure en acétate conduit au produit correspondant ; Rdt = 85%;

UV-visible (dans $H_2O$ à 4,4x$10^{-6}$ M) λ (ε) 640 (1,8 x $10^3$) ; 580 (6,4 x $10^3$) ; 556 ( 6,6 x$10^3$) ; 518 (1,3 x $10^4$); 422 (1,9 x $10^5$)(bande de Soret).

RMN $^1$H($CD_3COOD$) δ 9,48 (d, 6H, J = 5,8 Hz, protons 2,6-pyridine) ; 9,17 (s large, 6H, β-pyrrole) : 9,08 (d, 2H, J =4,8 Hz, β-pyrrole) ; 9,00 (d, 6H, J = 5,8 Hz, protons 3,5-pyridine) ; 8,27 (d, 2H, J = 8,3 Hz, protons 2,6-phényl) ; 7,51 (d, 2H, J = 8,3 Hz, protons 3,5-phenyl) ; 4,91 (s large, 9H, N-Me-4-pyridyl) ; 4,53 (m, 2H, $OCH_2$) ; 3,87 (m, 2H, $NCH_2$) ; 3,41 (s, 9H, $-NMe_3$) ; 2,63 (m, 2H, $-CH_2-$).

*r) Pentaacétate de cis-5,10-bis[4-N-trimethyl-3-aminopropyloxy)phenyl]-15,20-bis (N-methyl-4-pyridiyl)-porphyrine de manganese (III).*

En suivant le même mode opératoire que pour l'exemple (1h), nous obtenons à partir de composé (j) la porphyrine métallée avec le manganèse et méthylée sur les noyaux pyridiniques et portant deux bras 3-triméthylaminopropyloxy- en para des noyaux phényle.

Produit isolé = 0,015 g ; Rdt = 65 %.

UV-visible (dans $H_20$ à 9,3 x $10^{-6}$ M) λ (ε) 600 (6,9 x $10^3$) ; 564 (1,1 x $10^4$) ; 467 (8,7 x $10^4$) (bande de Soret).

EXEMPLE 4 : Dérivés (amino-butyryl)aminophénylique de porphyrine.

*s) 5-[4-(N-Boc-aminobutyryl)aminophenyl]-10,15,20-tris-(4-pyridyl)-porphyrine.*

Afin de synthétiser ce dérivé, un amino-acid du type N-Boc-$(CH_2)_n$-COOH est à préparer selon la méthode décrite dans le paragraphe suivant, ici n = 3.

*Acide N-Boc-4-aminobutyrique :*

A une suspension de 0,4 g (0,004 mole) d'acide 4-amino-butyrique, de 0,155 g (0,004 mole, 1 eq.) d'oxyde de magnésium et de 4 ml de soude 1M dans un mélange dioxane/eau (6/1), sont ajoutés lentement 0,96 g (0,0044 mole, 1,1 eq.) de dicarbonate de di-tert-butyle. Le mélange est agité pendant 20 h à température ambiante. Après filtration, le résidu es lavé à l'eau. Le filtrat est concentré, puis on ajoute l'eau. Aptès un lavage à l'éther, la phase aqueuse est acidifiée jusqu'à pH = 2-3 avec de l'acide acétique à 10 % puis extraite à l'acétate d'éthyle. La phase organique est séchée sur sulfate de sodium et évaporée à sec. L'huile orange obtenue est triturée avec l'hexane puis séchée sous vide à 60 ° C. Le produit obtenu est sous forme de solide blanc.

Produit isolé = 0,716 g ; Rdt = 90 %.

IR (pastille KBr) $\nu$ ($cm^{-1}$) 3343(NH et COOH) ; 1689 (large ; COOH et COOtBu).

Masse (DCI) : m/e = 204 ($M^+$ + 1) ; 221 ($M^+$ + 18).

RMN $^1H$ ($CDCl_3$) $\delta$ 11,34 (m, 1H, COOH) ; 3,14 (t dédoublé, 2H, J = 6,75 Hz, $CH_2N$) ; 2,36 (t, 2H, J = 6,8 Hz, $CH_2CO$) ; 1,79 (quintuplet, 2H, J = 6,8 Hz, -CH2-) ; 1,41 (s, 9H, tBu).

A l'aide de ce dérivé, la porphyrine (s) peut se préparer de la façon suivante :

A 0,063 g (0,31 mmole, 3,1 eq.) d'acide N-Boc-4-aminobutyrique dans 6 ml de dichlorométhane anhydre, maintenue dans un bain de glace, on ajoute 0,050 ml (0,36 mmole ; 4 eq.) de triéthylamine, puis 0,029 ml (0,3 mmole ; 3,3 eq.) de chloroformiate d'éthyle et on laisse le mélange agiter à 0 ° C pendant 30 mn. Le mélange est ensuite évapore à sec, puis repris dans 6 ml de dichlorométhane sec et refroidi dans un bain de glace. On ajoute 0,050 ml (0,36 mmole) de méthylamine, puis 0,066 g (0,1 mmole) de la porphyrine de l'exemple (1b).

Le mélange est agité à 0 ° C pendant 1 h, puis 2 h à température ambiante. Le solvant est ensuite évaporé à sec et le résidu est lavé abondamment à l'eau distillée.

Le résidu est repris dans du dichlorométhane. Cette solution est lavée avec du bicarbonate de sodium à 5 % (2 fois), puis à l'eau distillée (3 fois). La phase organique est séchée sur sulfate de sodium puis évaporée à sec. Le produit obtenu est presque pur, cependant, une purification sur une colonne sèche d'alumine permet l'obtention d'un produit très pur (éluant : $CH_2Cl_2$/EtOH, 99,5/0,5). Produit isolé = 0,066 g ; Rdt = 77 %.

Masse ($FAB^+$) : m/e = 819 ($M^+$ + 1) ; fragments : 763 (M-tBu) ; 719(M-COOtBu) ; 633 (M-C($CH_2$)-$_3$NHCOOtBu).

UV-visible (dans $CHCl_3$ à 2,04 x $10^{-6}$ M) λ (ε) 642 (2,9 x $10^4$) ; 586 (5,2 x $10^4$) ; 548 (6,8 x $10^4$) ; 512 (1,6 x $10^5$) ; 418 (1,6 x $10^6$)(bande de Soret).

IR (pastille KBr) $\nu$ ($cm^{-1}$) : 3319 (NH) ; 1689 (C=O) ; 1593 (Ar).

RMN $^1H$ ($CDCl_3$) $\delta$ 9,04 (m, 6H, protons 2,6-pyridine) ; 8,97 (d, 2H, J = 4,9 Hz, $\beta$-pyrrole) ; 8,84 (s, 4H, $\beta$-pyrrole) ; 8,81 (d, 2H, J = 4,9 Hz, $\beta$-pyrrole); 8,16 (d, 6H, J = 5,9 Hz, protons 3,5-pyridine) ; 8,08 (m, 4H, protons phenoxy)4) ; 4,27 (m, 2H, $CH_2N$); 2,57 (t, 2H, J = 6,5 Hz, $CH_2C$=O) ; 2,02 (m, 2H, -$CH_2$-) ; 1,53 (s, 9H, tBu) ; -2,90 (s, 2H, NH pyrrole).

*t) Acétate de 5-[4-(N-Boc-aminobutyryl)aminophenyl]-10,15,20-tris -(4-pyridyl)-porphyrine de manganèse (III).*

La métallation de la porphyrine de l'exemple (s) avec de l'acétate de manganèse dans du DMF selon la méthode déjà décrite dans l'exemple (1g) permet d'obtenir la porphyrine de manganèse attendue.

Produit isolé : m = 0,024 g ; Rdt = 70 %.

UV-visible (dans $CHCl_3$ à 2,15 x $10^{-6}$) λ (ε) 613 (6,7 x $10^4$) ; 578 (7,4 x $10^4$) ; 475 (8,9 x $10^5$) (bande de Soret).

*u) 5-[4-(aminobutyry)aminophenyl]-10,15,20-tris(4-pyridyl)-porphyrine*

Une solution de 0,027 g (0,03 mmole) de la porphyrine (s) dans 14 ml d'une solution à 25 % d'acide trifluoroacétique dans le dichlorométhane est agitée pendant 1 h à température ambiante. Le solvant est ensuite évaporé à sec. Au résidu obtenu est ajouté un large excès d'éther anhydre et une forte agitation est maintenue jusqu'à formation d'un précipité abondant. Après filtration, le précipité est dissous dans l'eau, puis neutralisé avec du bicarbonate de sodium à 5 %. La phase aqueuse ainsi obtenue est extraite au dichlorométhane (3 fois). Les phases organiques sont rassemblées, séchées sur sulfate de magnésium, puis évaporées à sec. Le produit est purifié par une précipitation à l'aide d'un mélange dichlorométhane/hexane.

Produit isolé = 0,020 g ; Rdt = 84 % ;

UV-visible (dans $CHCl_3$ à 3,62 x $10^{-6}$M) λ (ε) 645 (1,1 x $10^4$) ; 588 (1,7 x $10^4$) ; 548 (2,2 x $10^4$) ; 513 (3,3 x $10^4$) ; 419 (6,5 x $10^5$) (bande de Soret).

IR (lames $CaF_2$) n($cm^{-1}$) 3200 (NH large) ; 1629 (C=O large).

Masse ($FAB^+$) : m/e 719 $(M+1)^+$

RMN $^1$H ($CDCl_3$) δ 9,03 (d, 6H, J = 5,1 Hz, protons 2,6-pyridine) ; 8,83 (m, 8H, β-pyrrole) ; 8,15 (d, 6H, J = 5,1 Hz protons 3,5-pyridine) ; 7,97 (d, 2H, J = 8,1 Hz, protons 2,6-amino-4-phenyl) ; 7,08 (d, 2H, J = 8,1 Hz, protons 3,5-amino-4-phenyl) ; 3,24 (m, 2H, $CH_2N$) ; 2,03 (m, 4H, $CH_2C=O$ et $-CH_2$) ; -2,90 (m, 2H, NH pyrrole).

*v) Pentaacétate de 5-[4-(triméthylaminobutyryl)aminophényl]-10,15,20-tris(N-méthyl-4-pyridyl)-porphyrine de manganèse (III).*

La métallation de la porphyrine (u) avec de l'acétate de manganèse dans le DMF suivie d'une méthylation avec de l'iodure de méthyle dans le DMF (selon la méthode déjà décrite pour l'exemple (1h) conduit après l'échange de contre-ions idodure en acétate à la métalloporphyrine correspondante.

Produit isolé : 0,021 g ; Rdt = 87 % ;

UV visible (dans $H_2O$ à 3,76 x $10^{-5}$M) λ (ε) 596 (1,3 x $10^3$) ; 560 (2,8 x $10^3$) ; 462 (2,8 x $10^4$) (bande de Soret).

Masse ($FAB^+$) : m/e 844.

EXEMPLE 5 : Préparation d'une molécule hybride métalloporphyrine-intercalant.

a) Bromure de $N^2$ -(4-éthoxycarbonyl-butyl) -9-méthoxy-ellipticinium.

Un mélange de 9-méthoxy-ellipticine*(0,102 g ; 0,37 mmole) et de 5-bromo-valérate d'éthyle (0.058 ml : 0,37 mmole ; 1 eq.) dans 2 ml de DMF anhydre est chauffé à 120°C sous agitation pendant 4 heures. L'agitation est maintenue pendant la nuit à température ambiante puis, on ajoute de l'éther au mélange. Après filtration, la poudre orange obtenue est lavée à l'éther puis séchée sous vide. Produit isolé = 0,156 g ; Rdt = 87%.

UV-visible ($CHCl_3$) λ (ε) : 406 (3,9 x $10^3$); 390 (4,15 x $10^3$); 348 (3,4 x $10^3$); 332 (6,5 x $10^3$)

RMN $^1$H (DMSO-d6 à 303 K) δ : 10,11 (s, 1H, $H_1$); 8,57 (d, 1H, J = 7,1 Hz, $H_3$); 8,43 (d, 1H, J = 7,1 Hz, H4); 7,82 (d, 1H, J = 1,9 Hz, $H_{10}$); 7,62 (d, 1H, J = 8,8 Hz,$H_7$); 7,33 (dd, 1H, J = 2,2 Hz, 8,7 Hz, $H_8$); 4,83 (t,2H, J = 7,1 Hz, $(CH_2)_{12}$); 4,16 (q, 2H, J = 7,1 Hz, $(CH_2)_{17}$); 4,02 (s, 3H, $CH_3O$); 3,44 (s, 3H, $(CH_3)_{11}$); 2,84 (s,3H,$(CH_3)_5$); 2,53 (t,2H,J = 7,3 Hz, $(CH_2)_{15}$); 2,16 (m,2H,$(CH_2)_{13}$) ; 1,73 (m,2H,$(CH_2)_{14}$) ; 1,28 (t,3H,J = 7,1 Hz, $(CH_3)_{18}$). Les atomes de carbone du bras ont été numérotés de 12 à 18 en partant de l'azote 2 de la 9-méthoxy-ellipticine (le méthyle de la partie ester ayant le numéro 18).

b) chlorure de $N^2$-(4-carboxybutyl)-9-méthoxy- ellipticinium

0,148 g (0,3 mmole) d'ester (a) dans 14 ml d'acide chlorhydrique 1N sont chauffés au reflux sous agitation pendant 3 heures. L'agitation est maintenue pendant toute la nuit à la température ambiante. Le mélange est ensuite évaporé à sec; le résidu est repris dans du méthanol et on fait précipiter le produit par l'éther sous forme d'une poudre orange. Après filtration, le produit est séché sous vide. Produit isolé = 0,125 g : Rdt = 99%. La présence d'ions chlorure a été vérifiée par un test avec le nitrate d'argent.

* Ce composé et son activité ont été notamment décrits dans J. Le Men et coll., Rev. Europ. Etud. Clin. et Biol. 1970, XV, 534-538.

UV-visible ($CHCL_3$/MeOH) λ (ε) : 452 (1,29 x $10^3$); 386 (2,5 x $10^3$); 321 (1,8 x $10^4$)

Masse (DCI) =: $M^+$ = 377 Déc. 210 °C

RMN $^1H$ (DMSO-$d_6$ à 303 K) δ : 12,19 (1H,s,COOH); 10,22 (1H,s,$H_1$); 8,65 (d, 1H, J = 7,2 Hz, $H_3$); 8,59 (d, 1H, J = 7,2,Hz,$H_4$); 8,05 (d,1H,J = 2,3 Hz,$H_{10}$); 7,73 (d, 1H,J = 8,7 Hz,$H_7$); 7,44 (dd,1H,J = 8,7 Hz, J = 2,3 Hz, $H_8$); 4,84 (t,2H,J = 7,0 Hz, $(CH_2)_{12}$); 4,06 (s,3H,$CH_3$ O);3,46 (s,3H, $(CH_3)_{11}$); 3,43 (s,1H,NH); 2,97 (s,3H, $(CH_3)_5$; 2,45 (t,2H,J = 7,4 Hz, $(CH_2)_{15}$); 2,16 (m,2H, $(CH_2)_{13}$); 1,70 (m,2H,$(CH_2)_{14}$).

Le chlorure obtenu est dissous dans du méthanol. L'addition de résine essorée d'échangeurs d'ions de type Amberlite IRN 78 sous forme acétate (4 eq.) à cette solution suivie d'une agitation de 3 heures à la température ambiante permet, après filtration puis évaporation du solvant d'obtenir l'acétate correspondant. Le produit est ensuite purifié par précipitation dans un mélange méthanol/éther.

IR (lames NaCl) : $\nu_{CO}$ = 1656 $cm^{-1}$

UV-visible ($CH_3OH$) λ (ε) : 385 (5,7 x $10^3$); 318 (5,7 x $10^4$).

En suivant les mêmes procédés de synthèse, nous avons préparé les bromures de $N^2$-(2-éthoxycarbonyl-éthyl)-9-méthoxy-ellipticinium et de $N^2$-(5-éthoxycarbonyl-pentyl) -9-méthoxy-ellipticinium ainsi que leurs homologues porteurs d'une fonction acide:

*c) Chlorure de $N^2$-(2-carboxyéthyl)-9-méthoxy-ellipticinium*

Produit isolé : 0,096 g ; Rdt = 98 %.

UV-visible (dans MeOH à 2,2 x $10^{-5}$M) λ (ε) 410 (5,3 x $10^3$) ; 380 (8,0 x $10^3$) ; 305 (6,2 x $10^4$) ; 295 (6,5 x $10^4$) ; 274 (5,9 x $10^4$).

RMN $^1H$ (MeOH-$d_4$) δ 9,76 (s, 1H, $H_1$) ; 8,38 (d, 1H, J = 7,0 Hz, $H_3$) ; 8,31 (d, 1H, J = 7,0 Hz, $H_4$) ; 7,78 (d, 1H, J = 2,0 Hz, $H_{10}$) ; 7,54 (d, 1H, J = 8,8 Hz, $H_7$) ; 7,28 (d, 1H, J = 8,8 Hz, J = 2,0 Hz, $H_8$) ; 4,98 (m, 1H, $(CH_2)_{12}$) ; 4,02 (s, 3H, MeO) ; 3,25 (s, 3H, $Me_{11}$) ; 2,84 (m, 2H, $(CH_2)_{13}$) ; 2,79 (s, 3H, $Me_5$).

*d) Chlorure de $N^2$-(5-carboxypentyl)-9-méthoxy-ellipticinium.*

Produit isolé = 0,170 g : Rdt = 99 %.

UV-visible (dans MeOH à 3,3 x $10^{-5}$M) λ (ε) 440 (2,4 x $10^3$) ; 382 (6,1 x $10^3$) ; 3,13 (5,5 x $10^4$).

Masse (DCI) : $M^+$ = 391.

RMN $^1H$ (DMSO-$d_6$ à 294 K) δ 2,24 (s, 1H, COOH) ; 10,20 (s, 1H, $H_1$) ; 8,62 (d, 1H, J = 7,0 Hz, $H_3$) ; 8,56 (d, 1H, J = 7,0 Hz, $H_4$) ; 8,02 (d, 1H, J = 2,2 Hz, $H_{10}$) ; 7,72 (d, 1H, J = 8,8 Hz, $H_7$) ; 7,42 (dd, 1H, J = 8,8 Hz, J = 2,2 Hz, $H_8$) : 4,81 (t, 2H, J = 7,2 Hz, $(CH_2)_{12}$): 4,05 (s, 3H, MeO) ; 3,44 (s, 3H, $Me_{11}$) ; 2,95 (s, 3H, $Me_5$) ; 2,36 (t, 2H, J =7,0 Hz, $(CH_2)_{16}$) ; 2,13 (m, 2H, $(CH_2)_{13}$); 1,70 (m, 2H, $(CH_2)_{15}$); 1,47 (m, 2H, $(CH_2)_{14}$).

e) chlorure de 5-{4-[5-(9-méthoxy $N^2$-ellipticinium)-valéryl amino]-phényl}-10,15,20-tris-(4-pyridyl)porphyrine

A 0,054 g (0,13 mmole; 5,4 eq.) de (b) dans 2 ml de dichlorométhane anhydre, on ajoute 0,023 ml (0,16 mmole; 6,7 eq.) de la triéthylamine puis 0,021 ml (0,22 mmole; 9,1 eq.) de chloroformiate d'éthyle et on laisse le mélange sous agitation à température ambiante pendant 30 mn. Le mélange est ensuite évaporé à sec puis repris dans 2 ml de dichlorométhane anhydre. On ajoute 0,023 ml (0,16 mmole) de la triéthylamine puis 0,015 g (0,024 mmole) de la porphyrine de l'exemple 1 b) et on porte le mélange au reflux pendant 4 heures. L'avancement de la réaction est suivi par CCM. On laisse ensuite le mélange revenir à la température ambiante puis, le solvant est évaporé à sec. Le résidu repris par le dichlorométhane est purifié sur plaque de silice (éluant : EtOH/$CH_2Cl_2$ 20/80) à l'abri de la lumière. Le produit est repris par du méthanol et de l'acide acétique. Le filtrat est évaporé à sec, lavé à l'eau distillée puis séché sous vide. Produit isolé = 0,011 g; Rdt = 45%.

Masse (FD) : $M^+$-H = 991.

UV-visible ($CH_3OH$/AcOH : 99/1) λ (ε) : 318 (7,6 x $10^4$); 414 (5,2 x $10^4$); 508 (3,7 x $10^3$).

RMN $^1H$ (DMSO-d6 à 303 K) δ : 10,32 (s,1H,$H_1$); 9,16(d, 6H, J = 5,3 Hz, p-2,6-pyridine); 9,01 (m,8H,β-pyrrole); 8,73 (m,1H,$H_3$); 8,67 (m,1H,$H_4$); 8,38 (d,6H,J = 5,3 Hz,p-3,5-pyridine);8,21 (m,4H, amino-4-phényl);8,09 (s élargi, 1H,$H_{10}$);7,76 (d,1H,J = 8,7 Hz,$H_7$); 7,44 (d élargi, 1H,J = 8,7 Hz, $H_8$); 4,96 (m,2H-$(CH_2)_{12}$; 4,10 (m,1H,NH); 4,02 (s,3H,$CH_3$O); 3,53 (s,3H,$Me_{11}$); 3,01 (s,3H,$Me_5$);2,34 (m,2H,$(CH_2)_{15}$; 2,10 (m,4H,$(CH_2)_{13}$ et $_{14}$): -2,90 (s,2H,NH pyrrole).

f) tétraacétate de 5-{4-[5-(9-méthoxy-$N^2$-ellipticinium)-valérylamino]phényl}-10,15,20-tris(N-méthyl-4-pyridyl)porphyrine

Le résidu de l 'étape (e) précédente est repris dans 3 ml de DMF sec, 0,021 ml (0,34 mmole, 10 eq.) d'iodure de méthyle est ajouté puis, on porte le mélange au reflux pendant 3 heures. On laisse le mélange sous agitation à la température ambiante pendant 15 heures. Le solvant est ensuite évaporé à sec et le résidu est lavé à l'eau plusieurs fois. Après filtration, le précipité est trituré avec l'acétone puis filtré et séché sous vide. Produit isolé = 0,041 g; Rdt = 76%.

UV-visible (MeOH) λ (ε) : 655 (2,19 x $10^3$); 592 (3,4 x $10^3$); 555 (5,0 x $10^3$); 519 (8,4 x $10^3$); 425 (1,00 x $10^5$), bande de Soret); 318 (5,9.$10^4$).

L'échange d'ions iodure en acétate s'effectue avec de bons rendements à partir d'une solution méthanolique de ce produit ajoutée à la résin essorée d'échangeurs d'ions de type Amberlite IRN 78 sous forme acétate. Après 2-3 heures d'agitation à la température ambiante, le mélange est filtré et le solvant évaporé à sec. Le résidu est trituré avec de l'acétone, filtré et séché sous vide (Rdt = 80%).

UV-visible (MeOH) λ (ε) : 652 (1,7 x $10^3$) ; 590 (1,5 x $10^3$) ; 545 (2,3 x $10^3$) ; 512 (5,3 x $10^3$) ; 415 (7,7 x $10^4$), bande de Soret); 318 (2,7 x $10^4$).

Masse ($FAB^+$ dans HCl) : 1091.

RMN $^1H$ (DMSO-d6 à 296 K) δ : 10,32 (s,1H,$H_1$) ; 9,16 (d, 6H,J = 5,1 Hz, p-2,6-pyridine) ; 9,00 (m,8H,β-pyrrole) ; 8,73 (m,1H,$H_3$) ; 8,61 (m,1H,$H_4$) ; 8,37 (d,6H,J = 5,1 Hz, p-3,5-pyridine) ; 8,24 (s,4H,amino-4-phényl) ; 8,03 (s, 1H,$H_{10}$) ; 7,73 (d,1H,J = 8,70 Hz,$H_7$) ; 7,42 (dd,1H,J = 8,70 Hz,J = 2,4 Hz,$H_8$) ; 4,98 (m,2H,$(CH_2)_{12}$ ; 4,01 (s,3H, $OCH_3$) ; 3,70 (s,3H,$Me_{11}$) ; 3,48 (s,9H,$CH_3$) ; 2,98 (s,3H, $Me_5$) ; 2,33 (m,2H,$(CH_2)_{15}$ ; 2,13 (m,4H,$(CH_2)_{13}$ et $_{14}$) ; 2,03 (s,3H,$CH_3COO^-$) ; -2,91 (s,2H,NH pyrrole).

Cette molécule peut être métallée de la même manière que la prophyrine correspondante selon l'exemple 1 g). On préfère toutefois utiliser la méthode suivante combinant les étape e) et f) du présent exemple.

g) méthode générale de synthèse de métalloporphyrines hydrosolubles liées à une molécule d'intercalant consistant en la 9-méthoxy-ellipticine.

A 0,070 g (0,17 mmole, 4,8 eq.) de 5(b) dans 3 ml de dichlorométhane anhydre (ceci est également applicable à 5(c) et 5(d), on ajoute 0,037 ml (0,26 mmole : 7,6 eq.) de triéthylamine, puis 0,035 ml (0,37 mmole : 10,6 eq.) de chloroformiate d'éthyle et on laisse le mélange agiter à la température ambiante pendant 30 mn. Le mélange est ensuite évaporé à sec, puis repris dans 3 ml de dichlorométhane sec. On ajoute 0,037 ml (0,26 mmole de triéthylamine, puis 0,022 g (0,35 mmole) de la prophyrine de l'exemple 1 b) et on porte le mélange au reflux pendant 4h. Le mélange est ensuite évaporé à sec, puis repris dans 3 ml de DMF anhydre. 0,050 ml (0,38 mmole, 11 eq.) de 2,4,6-collidine, puis 0,39 mmole (11 eq.) de sel de manganèse, de fer ou de zinc ($Mn(CH_3COO)_2$, $4H_2O$; $FeCl_2$, $4H_2O$; $Zn(CH_3COO)_2$, $2H_2O$) sont ajoutés à la solution. Le mélange est chauffé à 140°C pendant 3h, puis on le laisse agiter à la température ambiante pendant 15h. Le solvant est ensuite évaporé à sec. Le résidu est lavé à l'eau distillée, puis purifié sur une colonne sèche d'alumine neutre (éluant : $CH_2Cl_2$ puis $CH_2Cl_2$/MeOH).

La métalloporphyrine ainsi obtenue est reprise dans 3 ml de DMF anhydre. L'iodure de méthyle (10 eq.) est ajouté à la solution. On porte le mélange au reflux pendant 3h, puis on le laisse agiter pendant 15 h à la température ambiante. Le solvant est évaporé à sec et le résidu repris dans du méthanol. L'addition de résine essorée d'échangeurs d'ions de type Amberlite IRN 78, sous forme acétate (4 eq.) à cette solution, suivie d'une agitation de 3h à la température ambiante, permet, après filtration puis évaporation du solvant, d'obtenir l'acétate correspondant. Le résidu est ensuite lavé au dichlorométhane, puis recristallisé dans un mélange méthanol/acétone. Les rendements et les données physico-chimiques des différentes molécules hybrides sont décrits ci-après.

Les rendements et les données physico-chimiques des différentes molécules hybrides sont décrits ci-après.

Molécules hybrides "intercalant-métalloporphyrine "résultant du couplage entre la porphyrine de l'exemple (1b) et l' ellipticinium de l'exemple (5b) (longueur du bras reliant les deux entités est de 7 chainons" Rdt = 60-80%.

UV-visible ($H_2O$) λ (ε)

(Mn) : 565 (4,8 x $10^3$); 465 (3,7 x $10^4$), bande de Soret); 316 (4,7.$10^4$).

bande de Soret; 3,15 (6,1 x $10^4$).

Masse ($FAB^+$) : pour (Mn) : 1182

* *Molécules hybrides "intercalant−métalloporphyrine" résultant du couplage entre la porphyrine de l'exemple (1b) et l'ellipticinium de l'exemple (5d). (longueur de bras reliant les deux entités est* de 8 chaînons).

*Pentaacétate de 5−{4−[6−(9−méthoxy−$N^2$−ellipticinium)−pentanoyl]amino−phényl}−10,15,20−tris−(N−méthyl−4−pyridyl)−porphyrine de manganèse (III).*

Rdt = 79 %

UV-visible ($H_2O$ à $1{,}86 \times 10^{-5}$ M) λ (ε) 600 ($4{,}3 \times 10^3$); 564 ($7{,}4 \times 10^3$); 464 ($1{,}6 \times 10^4$) (bande de Soret); 314 ($5{,}6 \times 10^4$).

* *Molécules hybrides "intercalant−métalloporphyrine" résultant de couplage entre la porphyrine de l'exemple 3(o) et l'ellipticinium de l'exemple (5b) longueur de bras reliant les deux entités est de 11 chaînons).*

- *La métalloporphyrine de manganèse (III) (N−methyl−4−pyridyl)correspondante :*

Rdt = 65 %.

UV-visible ($H_2O$ à $1{,}1 \times 10^{-5}$ M) λ (ε) 596 ($1{,}9 \times 10^3$) ; 564 ($3{,}6 \times 10^3$) ; 464 ($3{,}5 \times 10^4$) (bande de Soret) ; 314 ($2{,}3 \times 10^4$).

* *Molécules hybrides résultant du couplage entre la porphyrine de l'exemple 4(u) et l'ellipticinium (5b)(longueur du bras reliant les deux entités est de 12 chaînons) :*

- *La métalloporphyrine de manganèse (III) (N−méthyl−4−pyridyl)correspondante :*

Rdt. = 47 %.

UV-visible (MeOH à $1{,}1 \times 10^{-5}$ M) λ (ε) 598 ($3{,}6 \times 10^3$) ; 556 ($1{,}3 \times 10^4$) ; 421 ($2{,}8 \times 10^5$) ; 315 ($5{,}7 \times 10^4$).

* *Molécules hybrides résultant du couplage entre la métalloporphyrine de l'exemple 3(o) et de l'éllipticinium de l'exemple (5d)(longueur du bras reliant les deux entités est de 12 chaînons) :*

- *La métalloporphyrine de manganèse (III)(N−méthyl−4−pyridyl) :*

Rdt. = 44 %

UV-visible (H2O à $5{,}3 \times 10^{-6}$ M) λ (ε) 620 ($5{,}2 \times 10^3$); 674 ($8{,}5 \times 10^3$); 466 ($7{,}4 \times 10^4$) (bande de Soret); 315 ($7{,}5 \times 10^4$).

Pour chacun de ces composés on n'observe aucun point de fusion en-des sous de 240 °C.

Activités biologiques des composés préparés.

L'activité cytotoxique a été déterminée sur des cellules leucémiques murines de type L1210 selon une méthode déjà décrite (par C. Paoletti. S. Cros et coll., Chem. Bio. Interact., 25, 45 (1979). L'effet sur la croissance cellulaire est exprimé en termes de dose inhibant 50% de la croissance cellulaire ($DI_{50}$). Seules les valeurs des $DI_{50}$ inférieures à 2 uM peuvent être considérées comme significatives sur le plan biologique.

Les résultats de cytotoxicité sont rassemblés dans le tableau 1.

Les produits les plus intéressants de cette série sont les dérivés hydrosolubles du manganèse décrits dans les exemples, avec un, deux ou trois groupes pyridinium et un, deux ou trois groupes phényle portant une fonction amine ou alcool, permetant ainsi d'accrocher par l'intermédiaire d'un bras cette molécule à une autre entité ("vecteur") présentant une affinité pour les acides nucléiques. Ces dérivés métalloporphyriniques peuvent conduire à la synthèse de molécules hybrides cytotoxiques avec un vecteur (intercalant, oligonucléotides, oligopeptides, protéines ou fragments de protéines) adapté à la cible biologique (cellules tumorales, virus ...). Nous avons décrit de tels exemples (intercalants) dans l'exemple 5.

Par ailleurs, ces molécules cytotoxiques présentent une activité nucléase sur de l'ADN in vitro. Cette activité a été mise en évidence en étudiant les coupures sur la forme superenroulée (forme I)- de l'ADN de bactériophage φx 174 (voir tableau 2).

L'efficacité de coupure avec le composé (1h), possédant trois groupes pyridinium est très nette. En effet il ne reste que 13% de la forme I au bout de 2 mn en présence de 250 nM de métalloporphyrine et 5 uM d'hydrogénopersulfate de potassium. $KHSO_5$. Il s'agit là de coupures simples brins, puisque la forme II (ADN duplex circulaire avec une coupure) s'accumule avant de conduire à la forme III (ADN duplex linéaire). Les conditions expérimentales et les détails de la méthode d'analyse sont indiqués dans la référence Fouquet et coll. (cf. page 1).

Activités biologiques des molécules hybrides.

Nous avons choisi comme molécule hybride une métalloporphyrine (I) reliée à un intercalant de la série pyrido-carbazole comme vecteur, à savoir la 9-méthoxy-ellipticine.

La molécule hybride (5f) dans laquelle le métal central est le manganèse est caractérisée par une $DI_{50}$ de 0,58 μM, voir tableau 1.

Il faut noter que la molécule de l'exemple 5 présente toujours une activité nucléase importante, bien que plus faible que l'exemple 1(h). La quasi-complète transformation de la forme I en forme II est obtenue à

une concentration de 4 µM en composé (5f).

Les molécules hybrides selon l'invention peuvent être considérées comme les premiers modèles biologiquement actifs de la bléomycine, médicament antitumoral agissant par dégradation oxydante de l'ADN en présence de sels métalliques (Sausville et coll., Biochemistry, 17, 2740 (1978).

En conclusion, les composés de l'invention présentent à la fois une activité cytotoxique sur des cellules tumorales et une capacité à couper les acides nucléiques in vitro.

Comme type de vecteur capable de moduler l'affinité ou l'interaction de ces molécules cytotoxiques vis-à-vis des acides nucléiques on peut citer des intercalants, cas décrits ci-dessus, des oligonucléotides, oligo- ou polypeptides. ou des polyamines...).

Il s'agit là d'une nouvelle série originale de molécules cytotoxiques ayant pour cible les acides nucléiques. La large gamme des vecteurs associables aux composés de formule I permet d'envisager une activité cytotoxique sur des cellules de type très différent : cellules tumorales, virus ...

Les composés de l'invention sont donc utilisables en thérapeutique, notamment comme antitumoraux, antileucémiques et antiviraux. L'invention a aussi pour objet les compositions pharmaceutiques contenant ces composés ainsi qu'un véhicule ou excipient pharmaceutiquement acceptable.

Tableau 1

| Effets in vitro sur des cellules L1210 de quelques composés selon l'invention | |
|---|---|
| Composé de l'exemple | $DI_{50}$ (µM) |
| 1 h)(Mn) | 0,54 |
| 2 n)(Mn) | 0,68 |
| 5 b) | >23 |
| 5 f)(Mn) | 0,58 |
| 5 g)(Mn) | 0,84 |

Tableau 2 - Observation par électrophorèse sur gel d'agarose des effets endonucléases

| Composé | Conditions |
|---|---|
| | 1 µM en métalloprophyrine<br>5 µM en $KHSO_5$<br>temps de pré-incubation (ADN+métallo-porphyrine): a) 20 mn, b) 1 mn temps d'incubation (avec oxydant) : 2mn |

| Produit | Formes | I | II | III |
|---|---|---|---|---|
| (ADN témoin) | | 85% | 15% | - |
| 1 h)(Mn) (250 nM)[a] | | 13% | 78% | 9% |
| 1 h)(Mn) (250 nM)[b] | | 63% | 37% | - |
| 1 h)(Mn) (1 µM)[a] | | - | 59% | 41% |
| 5 f)(Mn) (1 µM)[a] | | 32% | 65% | 3% |
| 5 f)(Mn) (4 µM)[a] | | - | 71% | 10% |

## Revendications

**1.** Molécule hybride résultant du couplage d'un vecteur présentant une affinité pour les acides nucléiques - choisi parmi la 9-méthoxy-ellipticine, les oligonucléotides, oligopeptides, protéines et fragments de protéines - et d'un dérivé de métalloporphyrine de formule générale

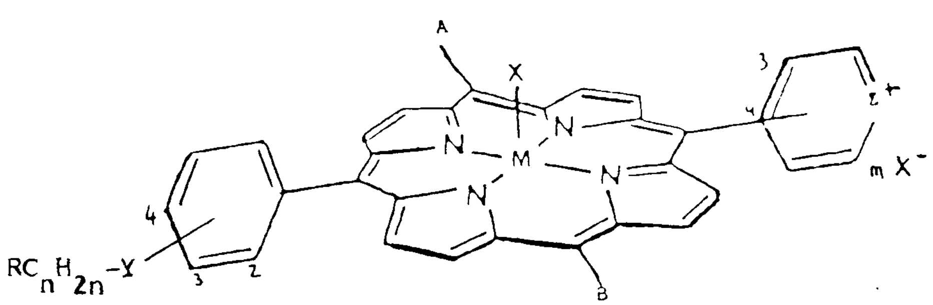

I

dans laquelle A et B représentent chacun

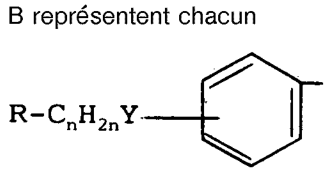

ou

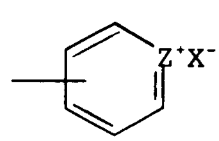

et $Z^+$ représente $N^+-R_1$ ou $C-N^+R_1R_2R_3$, $R_1$ étant un groupe aliphatique droit ou ramifié en $C_1$ à $C_{10}$, et $R_2$ et $R_3$ étant chacun un atome d'hydrogène ou un groupe aliphatique droit ou ramifié en $C_1$ à $C_{10}$, R représente un groupe $NH_2$, OH, COOH ou $-N(R_1)^+{}_3$ ou un atome d'halogène,
n est 0 ou un entier de 1 à 10, le groupe alkylène correspondant pouvant être droit ou ramifié,
M représente Mn,
$X^-$ représente l'anion d'un acide carboxylique pharmaceutiquement acceptable, m étant un entier de 1 à 5,
et Y représente une liaison ou, lorsque n est différent de 0, un radical -O-, -CO- ou -CONH-.

**2.** Molécule hybride selon la revendication 1 dans laquelle ledit dérivé de métalloporphyrine est relié audit vecteur par une fonction amine ou alcool greffée sur un groupe phényle de la métalloporphyrine.

**3.** Molécule hybride selon la revendication 1 dans laquelle ledit vecteur est la 9-méthoxy-ellipticine.

**4.** Composition pharmaceutique contenant un complexe selon la revendication 1 associé à un excipient pharmaceutiquement acceptable.

**5.** Utilisation de molécules hybrides selon la revendication 1, pour la préparation d'un médicament antitumoral, antileucémique ou antiviral.

## Claims

**1.** Hybrid molecule resulting from the coupling of a vector having an affinity for nucleic acids - selected from 9-methoxy-ellipticine, the oligonucleotides, oligopeptides, proteins and protein fragments - and of a metalloporphyrine derivative having the general formula

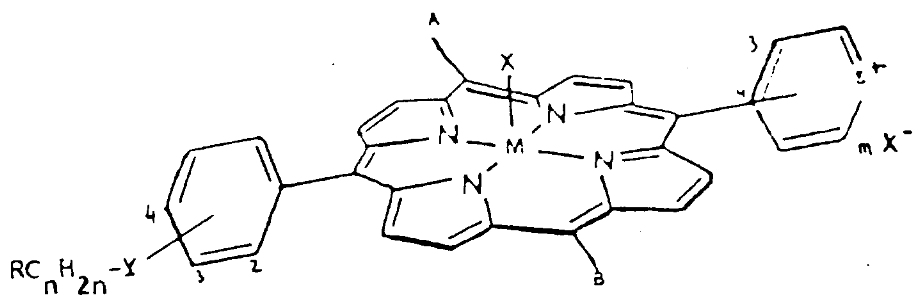

I

in which A and B each represent

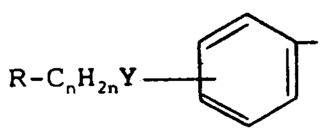

or

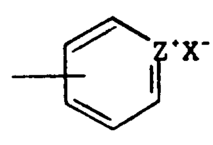

and $Z^+$ represents $N^+-R_1$ or $C-N^+R_1R_2R_3$, $R_1$ being a straight or branched aliphatic group of $C_1$ to $C_{10}$, and $R_2$ and $R_3$ each being a hydrogen atom or a straight or branched aliphatic group of $C_1$ to $C_{10}$, R represents an $NH_2$, OH, COOH or $-N(R_1)^+{}_3$ or a halogen atom,
n is 0 or an integer from 1 to 10, in which case the corresponding alkylene group may be straight or branched,
M represents Mn,
$X^-$ represents the anion of a pharmaceutically acceptable carboxylic acid, m being an integer from 1 to 5, and Y represents a bond or, when n is other than 0, an -O-,-CO- or -CONH- radical.

**2.** Hybrid molecule according to claim 1, in which the metalloporphyrine derivative is linked to the vector by an amine or alcohol function grafted to a phenyl group of the metalloporphyrine.

**3.** Hybrid molecule according to claim 1, in which the vector is 9-methoxy-ellipticine.

4. Pharmaceutical compound containing a complex according to claim 1 associated with a pharmaceutically acceptable excipient.

5. Use of hybrid molecules according to claim 1 for the preparation of an antitumour, antileukaemic, or antiviral medicament.

**Patentansprüche**

1. Hybrid-Molekül, wie es beim Kuppeln eines Vektors, der eine Affinität für die Nucleinsäuren - ausgewählt aus der Gruppe 9-Methoxy-ellipticin, den Oligonucleotiden, Oligopeptiden, Proteinen und Proteinfragmenten - aufweist, mit einem Metallporphyrin-Derivat der allgemeinen Formel (I) erhalten wird

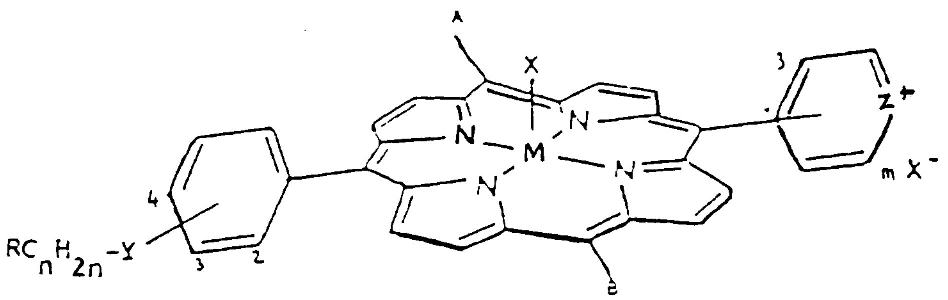

(I)

worin bedeuten:
A und B jeweils

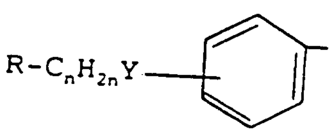

oder

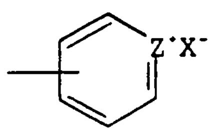

| | |
|---|---|
| $Z^+$ | $N^+$-$R_1$ oder C-$N^+R_1R_2R_3$, worin R für eine gerade oder verzweigte aliphatische $C_1$-$C_{10}$-Gruppe und $R_2$ und $R_3$ jeweils für ein Wasserstoffatom oder eine gerade oder verzweigte aliphatische $C_1$-$C_{10}$-Gruppe, |
| R | eine $NH_2$-, OH-, COOH- oder -$N(R_1)^+{}_3$-Gruppe oder ein Halogenatom, |
| n | die Zahl 0 oder eine ganze Zahl von 1 bis 10, wobei die entsprechende Alkylengruppe gerade oder verzweigt sein kann, |
| M | Mn, |
| $X^-$ | das Anion einer pharmazeutisch akzeptablen Carbonsäure, |
| m | eine ganze Zahl von 1 bis 5 und |
| Y | eine Bindung oder dann, wenn n von 0 verschieden ist, einen -O-, -CO- oder -CONH-Rest. |

2. Hybrid-Molekül nach Anspruch 1, in dem das genannte Metallporphyrin-Derivat über eine Aminfunktion oder Alkoholfunktion, die auf eine Phenylgruppe des Metallporphyrins aufgepfropft ist, mit dem genannten Vektor verbunden ist.

3. Hybrid-Molekül nach Anspruch 1, in dem der genannte Vektor das 9-Methoxy-ellipticin ist.

4. Pharmazeutische Zusammensetzung, die einen Komplex nach Anspruch 1 enthält, der mit einem pharmazeutisch akzeptablen Exzipienten assoziiert ist.

5. Verwendung der Hybrid-Moleküle nach Anspruch 1 zur Herstellung eines Antitumor-, Antileukämie- oder Antiviren-Arzneimittels.